# EUROPEAN PATENT SPECIFICATION

(11) **EP 2 074 988 B1**
(45) Date of publication and mention of the grant of the patent: **14.03.2018**
(21) Application number: 07025176.4
(22) Date of filing: 28.12.2007
(51) Int. Cl.: A61K 9/16, A61K 9/70

(54) **Sustained release composition and manufacturing method thereof**
Zusammensetzung mit verzögerter Freisetzung und Herstellungsverfahren dafür
Composition à libération prolongée et son procédé de fabrication

(43) Date of publication of application: 01.07.2009
(73) Proprietor: Industrial Technology Research Institute, 31040 Hsinchu (TW)
(72) Inventor: Lu, Jui-Mei, Hsinchu County 310 (TW); Liu, Chia-Wen, Taoyuan County 326 (TW); Lai, Po Hong, Pingjhen City Taoyuan County 324 (TW); Lin, John, Jiang, Hann, Keelung City 201 (TW); Li, Chiao Pin, Kaohsiung City 807 (TW); Cheng, Sung, En, Kaohsiung County (TW); Lo, Yo Wen, Chiayi County 608 (TW); Sheu, Ming-Thau, Sijhih City Taipei County 221 (TW); Lin, Min-Ying, Hsinchu City 300 (TW)
(74) Representative: Fuchs Patentanwälte Partnerschaft mbB

(56) References cited:
- WO-A-96/28143
- WO-A-97/09985
- WO-A-2004/089291
- WO-A-2005/049090
- WO-A-2005/070332
- WO-A-2008/026894
- US-A1- 2002 009 493
- DIAZ R V ET AL: "Effect of surfactant agents on the release of <125>I-bovine calcitonin from PLGA microspheres: in vitro -- in vivo study" JOURNAL OF CONTROLLED RELEASE, ELSEVIER, AMSTERDAM, NL, vol. 43, no. 1, 3 January 1997 (1997-01-03), pages 59-64, XP004069874 ISSN: 0168-3659
- NAHATA T ET AL: "Optimization of formulation variables for the development of long acting microsphere based depot injection of olanzapine." JOURNAL OF MICROENCAPSULATION SEP 2008, vol. 25, no. 6, September 2008 (2008-09), pages 426-433, XP009108033 ISSN: 1464-5246

## Description

### BACKGROUND OF THE INVENTION

### Field of the Invention

The present invention relates to a sustained release system, and in particular relates to a sustained release composition containing release rate determined agent.

### Description of the Related Art

The desirability of coating medical devices such as, surgical implants, sutures and wound dressings with pharmaceutical agents is well known. Such coated devices provide a means for locally delivering pharmaceutical or therapeutic agents at the site of medical intervention to treat a variety of diseases. For example, surgical implants or sutures coated with antibiotics can provide local delivery of antibiotics directly to an implantation or suture site, thereby decreasing the onset of infection following the surgical intervention.

Thus, there is an increasing interest in developing a drug delivery system which is both safe and which provides a high biological availability of the drug, i.e. to maximize pharmaceutical activity of known drugs as well as to minimize the side effects thereof. Due to their uniform release rate during a given time period and the non-toxic property of degradation products, biodegradable polymers have been widely investigated as drug carriers. Biodegradable polymer drug carriers are especially useful for delivering drugs requiring continuous and sustained release with a single administration, e.g. peptide or protein drugs, which should be administered daily because of quick loss of activity in the body.

However, the traditional drug release curve includes three phases with an initial release phase, a lag phase, and a secondary release phase, wherein the drugs are only released at initial and secondary release phases. Thus, at the lag phase, it is necessary to combine an oral administration with the local administration to achieve a desired therapeutic goal, with the additional oral administration increasing costs and causing inconvenience. To overcome the above problems, a novel sustained release composition and manufacturing method thereof are needed.

US 2002/009493 A1 refers to a method for preparing a PLGA delivery system which releases active agents in dependence of proportions of LA to GA by oil-in-oil emulsion-solvent extraction. A method is disclosed which offers protection and maintains stability for an active ingredient such as a tissue plasminogen activator, fibroblast growth factor or morphogenic protein-2 using a basic additive including magnesium or zinc salts.

WO 2004/089291 A refers to a composition for tumor treatment including a fast-release and slow-release formulation of a drug and a pharmaceutically acceptable carrier. Formulations with micro- or nano-sized particles are disclosed. The compositions may contain PLGA to encapsulate paclitaxel or doxorubicin by an emulsion process.

WO 96/28143 A concerns polypeptide-containing parenteral pharmaceutical forms of administration in the form of microparticles. The respective method uses a triblock ABA (A: PLGA, B: PEG) polymer to encapsulate the polypeptide by a W/O/W emulsion process. The method comprises the addition of additives into a water phase to prevent an aggregation and decrease the release rate of the polypeptide.

WO 2005/049090 A refers to a composition containing olanzapine for transdermal administration. The compositions include olanzapine or a pharmaceutically acceptable salt thereof, a pressure sensitive adhesive, and an excipient, such as a permeation enhancer and/or a solubilizer of olanzapine. The skin-contacting layer compositions can be prepared by combining the pressure sensitive adhesive copolymer, drug, permeation enhancer, and optional additives with an organic solvent.

WO 97/09985 A refers to a transdermal administration of olanzapine and pharmaceutically acceptable salts thereof. Permeation enhancer are disclosed that may include PGML, which is a mixture of propylene glycol monolaurate, propylene glycol dilaurate, and either propylene glycol, methyl laurate, or both.

WO 2005/070332 A discloses an implant which could control release of antipsychotic agents for 14 to 420 days by using different LA/GA ratio PLGA materials.

Diaz et. al. studies the effect of surfactant agents on the release of 1251-bovine calcitonin from DL,PLGA microspheres prepared by a double emulsion technique including Tween®-80 in a water phase and Span®-60 in an oil phase (Diaz et. al., Journal of Controlled Release 43 (1997) 59-64).

Zhang and Feng disclose paclitaxel-loaded nanoparticles of a modified poly(lactide)/vitamin E-TPGS-copolymer (PLA-TPGS) for cancer chemotherapy. The nanoparticles are produced by a modified solvent extraction/evaporation technique (Zhang, Z., Feng, S.-S., Biomaterials 27 (2006) 262-270).

### BRIEF SUMMARY OF INVENTION

The invention provides a sustained release composition, comprising a polymer, a bioactive agent, and a release rate determined agent, wherein the bioactive and the release rate determined agent are dispersed in the sustained release composition, and the release rate determined agent controls a release rate of the bioactive agent.

The invention further provides a method for manufacturing a sustained release composition, comprising: providing an oil phase comprising a bioactive agent, a polymer, and release rate determined agent; providing an aqueous phase comprising a surfactant; mixing the oil phase with the aqueous phase to form the sustained release composition having a controlled release effect.

The invention further provides a method for treating an animal, comprising administering a pharmaceutically effective amount of the sustained release composition of the invention.

A detailed description is given in the following embodiments with reference to the accompanying drawings.

### BRIEF DESCRIPTION OF DRAWINGS

The present invention can be more fully understood by reading the subsequent detailed description and examples with references made to the accompanying drawings, wherein:

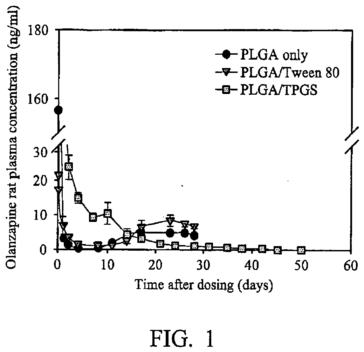

Fig. 1 shows the drug release curve of the sustained release compositions.

### DETAILED DESCRIPTION OF INVENTION

The following description is of the best-contemplated mode of carrying out the invention. This description is made for the purpose of illustrating the general principles of the invention and should not be taken in a limiting sense. The scope of the invention is best determined by reference to the appended claims.

The invention provides a sustained release composition, comprising a polymer, a bioactive agent, and a release rate determined agent, wherein the bioactive and the release rate determined agent are dispersed in the sustained release composition, and the release rate determined agent controls a release rate of the bioactive agent.

The sustained release composition may contain one or more of the bioactive agents, wherein the bioactive agent can be present in the oil phase at a concentration of 0.1-10% by weight, preferably, 2% by weight.

TPGS (d-alpha-tocopheryl polyethylene glycol succinate) is commercially available from Eastman Chemical Company as "Vitamin E-TPGS". Vitamin E-TPGS is a water soluble derivative of natural source vitamin E, and similar to an amphiphile. In the invention, various chemical derivatives of vitamin E-TPGS including ester and ether linkages of various chemical moieties are included within the definition of vitamin E-TPGS. Rather, it is dispersed in the oil phase to control the release of the bioactive agent. According to an important feature of the invention, the drug release curve of the sustained release composition of the invention does not have a lag phase. Thus, the bioactive agent can be continuously released, which obviates the need for using additional oral administration. In the invention, TPGS is present in as amount of about 1 to 50 % by weight of the total weight of the sustained release composition, preferably, 4 to 15 % by weight.

In one embodiment, the sustained release composition of the invention has a drug encapsulation rate exceeding 70%, preferably, 70% to 99.9%, a diameter exceeding 5 µm, preferably, 5 µm to 200 µm, most preferably 30 µm to 100 µm, and can continuously release the bioactive agent in the biological body in absence of the lag phase.

The sustained release compositions of the present invention can be applied systemically or topically. For example, the compositions can be administered by injection intramuscularly, intraperitoneally intravenously or subcutaneous. Additionally, the compositions can be in virtually any form, for example, lotions, ointments, creams, gels, drops, suppositories, sprays, liquids and powders. Conventional pharmaceutical carriers, aqueous, powder or oily bases, thickeners and the like may be incorporated.

The invention further provides a method for manufacturing a sustained release composition. The method comprises providing an oil phase; providing an aqueous phase comprising a surfactant; and mixing the oil phase with the aqueous phase to form an emulsion and removing solvents to form hardened microparticles, wherein the polymer is polylctide-co-glycolide (PLGA), the bioactive agent is olanzapine, and, wherein the oil phase contains a bioactive agent, a polymer, and a release rate determined agent.

As used herein, the term "oil phase" of the invention refers to the solution of the solvent, polymer, bioactive agent, and release rate determined agent, that will be mixed with an aqueous phase through emulsion process to provide the sustained release composition of the invention. The solvent of the oil phase includes, but are not limited to, methylene chloride, ethyl acetate, benzyl alcohol, acetone, acetic acid, propylene carbonate, dichloromethane, chloroform, 1,4-dioxane, dimethylformamide (DMF), dimethyl sulphoxide (DMSO), toluene, or tetrahydrofuran (THF).

The terms "release rate determined agent" of the invention refers to the solution of the surfactants which can control the release rate of the bioactive agent.

The terms "aqueous phase" refers to the solution of water and surfactants that is contacted with an oil phase through an emulsion process to provide the sustained release compositions of the present invention. The release rate determined agent are present at a concentration in the oil phase between 1 and 50% by weight. For example, they are present at a concentration between 4 to 15% by weight.

The surfactants of the invention include, but are not limited to, polyvinyl alcohol (PVA), NP-5, Triton X-100, Tween® 80, PEG 200-800, sodium dodecyl sulfate (SDS), alcohol ethoxylates, alkylphenol ethoxylates, secondary alcohol ethoxylates, fatty acid ester, or alkyl polygylcosides. The surfactants are present at a concentration in the aqueous between 0.1 and 10% by weight. For example, they are present at a concentration between 0.1 to 5% by weight.

In one embodiment, the oil phase is contacted with the aqueous phase to form an emulsion, wherein the emulsion comprises droplets of the oil phase dispersed in the aqueous phase. Solvent is subsequently removed from the emulsion droplets to form hardened microparticles. The solvent can be removed by evaporation, filtration, or extraction into an extraction liquid. For example, the extraction liquid may be water. The hardened microparticles may then be recovered from the aqueous phase and dried.

The emulsion is produced by stirring the organic and aqueous phases. In one embodiment, the emulsion is produced by use of a mixer, such as a static mixer. Alternatively, the emulsion is produced by use of turbulent mixing.

The emulsion process may be carried out at any temperature between the boiling point and freezing point of the components. In one embodiment, the temperature ranges from 0°C to 100°C, or between about 5°C to 75°C, or between about 15°C and about 60°C.

Additionally, cosolvents may be added to the oil phase. They are optionally used to promote solubility of the bioactive agent in the oil phase. In one embodiment, they include, but are not limited to, dimethyl sulfoxide, dimethyl formamide, n-methylpyrrolidinone, PEG 200, PEG 400, methyl alcohol, ethyl alcohol, isopropyl alcohol, and benzyl alcohol. The cosolvent may be present between 0 and 90% by weight of the solvent of the oil phase, or between 0 and 50% by weight of the solvent of the oil phase. The bioactive agent may be dissolved first in an appropriate volume of the cosolvent which is then added to the solvent of the oil phase, optionally dissolved with the polymer, so as to form a solution containing all the components of the oil phase. It should be noted that, a person of ordinary skill may adjust the volumes and order of addition to achieve the desired solution of bioactive agent and polymer. In one embodiment, the bioactive agent will be present in the oil phase at a concentration of 0.1 to 10% by weight. In another embodiment, the polymer will be present in the oil phase at a concentration of 0.1 to 20% by weight. For example, the polymer will be present in the oil phase at a concentration of 1 to 10% by weight.

The treatment is not necessary to combine an oral administration with the local administration to improve treatment effects, because the sustained release composition can continuously release bioactive agent.

The sustained release compositions of the invention may be suspended in any aqueous solution or other diluent for injection in a human or animal patient in need of treatment. Aqueous diluent solutions may further include a viscosity enhancer selected from the group consisting of sodium carboxymethylcellulose, sucrose, mannitol, dextrose, trehalose and other biocompatible viscosity enhancing agents.

### EXAMPLE

### Example 1: 50/50 PLGA microspheres contained 0, 7, 26, 42% vitamin-E TPGS

80 mg of olanzapine, 200 mg PLGA (LA/GA ratio = 50/50, M.W. = 43000) and distinct amount vitamin E-TPGS (0, 7, 26, 42% by weight) were co-dissolved in 5 mL of dichloromethane to form an oil phase. The oil phase was dropped into 1000 mL of cooled aqueous phase containing 0.1 % polyvinyl alcohol (PVA) and emulsified at 1000 rpm. The resulting o/w emulsion was agitated continuously for 3 h at room temperature. The microspheres were collected by centrifugation, washed with F68, water and freeze-dried. Then the microspheres were evaluated for particle size and drug encapsulated efficiency by Multisizer and High Performance Liquid Chromatography (HPLC). The results showed that the particle size were 68.4±28.3, 71.6±27.3, 91.0±35.8, 101.9±42.8 µm and the encapsulated efficiency were 77.7, 83.5, 85.9, 85.2% of the microspheres contained 0, 7, 26, 42% (w/w) vitamin E-TPGS, respectively.

### Example 2: 85/15 PLGA microspheres contained 0, 4, 15, 26% vitamin E-TPGS

320 mg of olanzapine, 800 mg PLGA (LA/GA ratio = 85/15, M.W. = 53000) and distinct amount vitamin E-TPGS (0, 4, 15, 26% by weight) were co-dissolved in 15 mL of dichloromethane to form an oil phase. The oil phase was dropped into 2000 mL of cooled aqueous phase containing 0.1 % polyvinyl alcohol (PVA) and emulsified at 1000 rpm. The methods of agitation, collection and analysis are as previously defined in Example 1. The results showed that the particle size were 40.out21.3, 58.8±28.4, 47.4±21.4, 62.9±25.9 µm and the encapsulated efficiency were 77.2, 74.7, 87.6, 83.7% of the microspheres contained 0, 4, 15, 26% (w/w) vitamin E-TPGS, respectively.

### Example 3: 50/50 PLGA microspheres contained a different aqueous phase

80 mg of olanzapine, 200 mg PLGA (LA/GA ratio = 50/50, M.W. = 43000) and 42 % (w/w) of vitamin E-TPGS were co-dissolved in 5 mL of dichloromethane to form an oil phase. The oil phase was dropped into 1000 mL of a cooled aqueous phase and then emulsified at 1000 rpm, wherein the cooled aqueous phase included 0.05 % PVA or 0.05% PVA containing 0.5% gelatin. The resulting o/w emulsion was agitated continuously for 3 h at room temperature. The microspheres were collected by centrifugation, washed with F68, water and free-dried. The analysis methods of the particle size and drug encapsulated efficiency are as previously defined above. The results showed that the particle size were 108.7±37.3, 85.5±31.4 µm and the encapsulated efficiency were 74.1, 73.1% of the microspheres contained 0.05 % PVA without and with 0.5% gelatine, respectively.

### Example 4: 85/15 PLGA microspheres contained a different oil phase

320 mg of olanzapine, 800 mg PLGA (LA/GA ratio = 85/15, M.W. = 43000) and 15 w/w% of vitamin E-TPGS were co-dissolved in 15 mL of dichloromethane to form an oil phase. In this Example, dichloromethane included pure dichloromethane (Formulation #1), dichloromethane containing ethanol (DCM : ethanol = 2 : 1, Formulation #2), and dichloromethane containing acetone (DCM : acetone = 2 : 1, Formulation #3), respectively. The oil phase was dropped into 2000 mL of cooled aqueous phase containing 0.1 % PVA and emulsified at 1000 rpm. The methods of agitation, collection and analysis are as previously defined in Example 1. The results showed that the particle size were 76.2±31.4, 70.6±31.8, 70.6±31.8 µm and the encapsulated efficiency were 86.8, 89.7, 88.5% of the microspheres in the Formulation #1, #2, #3.

### Example 5: 85/15 PLGA microspheres contained a different LA/GA ratio

320 mg of olanzapine, 800 mg of PLGA and 15 % (w/w) vitamin E-TPGS were co-dissolved in 15 mL of dichloromethane to form an oil phase, wherein the PLGA had a different LA/GA ratio, which included 85/15 : 50/50 = 3 : 1 (Formulation #1), 85/15 : 50/50 = 1 : 3 (Formulation #2), and 75/20 : 50/50 = 4 : 1 (Formulation #3), respectively. The oil phase was dropped into 2000 mL of cooled aqueous phase containing 0.1 % PVA and emulsified at 1000 rpm. The methods of agitation, collection and analysis are as previously defined in Example 1. The results showed that the particle size were 62.2±32.0, 59.1±24.9, 41.1±19.1 µm and the encapsulated efficiency were 84.9, 84.2, 83.2% of the microspheres in the Formulation #1, #2, #3.

### Example 6: 75/25 PLGA microspheres

320 mg of olanzapine, 800 mg PLGA (LA/GA ratio = 75/25) and 4 % (w/w) of vitamin E-TPGS were co-dissolved in 15 mL of dichloromethane to form an oil phase. In this Example, the molecule weight of the PLGA was 30000 Da. and 21000 Da., respectively. The oil phase was dropped into 2000 mL of cooled aqueous phase containing 0.1 % PVA and emulsified at 1000 rpm. The methods of agitation, collection and analysis are as previously defined in Example 1. The results showed that the particle size were 43.5±18.8, 37.4±20.2 µm and the encapsulated efficiency were 80.8, 77.6% of the microspheres comprised of 30000,21000 Da. PLGA, respectively.

### Example 7: 85/15 PLGA microspheres

320 mg of olanzapine, 800 mg PLGA (LA/GA ratio = 85/15) and 4 % (w/w) of vitamin E-TPGS were co-dissolved in 15 mL of dichloromethane to form an oil phase. In this Example, the molecule weight of PLGA included 53000 and 27000 Da., respectively. The methods of agitation, collection and analysis are as previously defined in Example 1. The results showed that the particle size were 58.8±28.4, 43.9±20.6 µm and the encapsulated efficiency were 74.7, 77.0% of the microspheres comprised of 5300D, 27000 Da. PLGA, respectively.

### Example 8: different release rate determined agents formulations

320 mg of olanzapine, 800 mg PLGA (LA/GA ratio = 85/15, M.W. = 53000) and distinct release rate determined agent (Span® 80, Span® 85, oleic acid, PEG-PCL di-block copolymer, glyceryl tricaprylate, Pluronic®, Tween® 80) were co-dissolved in 15 mL of dichloromethane to form an oil phase. The oil phase was dropped into 2000 mL of cooled aqueous phase containing 0.1 % polyvinyl alcohol (PVA) and emulsified at 1000 rpm. The methods of agitation, collection and analysis are as previously defined in Example 1. The results showed that the particle size were 46.7±18.6, 40.3±15.5, 46.3±22.6, 55.7±27.3, 49.5±22.0, 68.1±30.9, 30.1±14.9 µm and the encapsulated efficiency were 86.9, 78.2, 79.4, 87.2, 69.5, 85.2, 75.5% of the microspheres comprise distinct release rate determined agent, respectively.

### Example 8: Drug release in vivo

Sprague-Dawley rats were selected for the evaluation of depot formulations because the size of their leg muscles facilitates dose administration and evaluation of the injection site.

The male Sprague-Dawley rats weight between 250 to 300 g. Rats were given a single injection with a 20- or 21-gauge needle into the biceps femoris. The dose volume varied with the concentration of the formulation but did not exceed 1 mL per injection. The rats were given 40 mg of olanzapine/kg body weight.

At each time point, a 0.1 mL blood sample was collected from the lateral tail vein into heparanized collection tubes. Blood samples were collected once prior to dose administration and at various time points after dose administration throughout the 50-day period. Typical time points are at 5 min, 10 min, 30 min, 1 hr, 2 hr, 4 hr, 6 hr, 1 d, 2 d, 4 d, 7 d,10 d, 14 d, 17 d, 21 d, 24 d, 28 d, 31 d, 35 d, 38 d, 42 d, 45 d, 50 d after dose administration. Plasma was harvested and plasma concentration of olanzapine was determined by HPLC/MS-MS.

Microsphere containing 15 % (w/w) vitamin E-TPGS prepared in Example 2, was administrated into rats by intramuscular injection. In the control groups, the TPGS of the microsphere was removed (PLGA only) or substituted for Tween 80 (PLGA/Tween 80). Referring to Fig. 1, microsphere containing TPGS continuously released olanzapine in the rats, and the drug release curve of microsphere did not have the lag phase.

While the invention has been described by way of example and in terms of the preferred embodiments, it is to be understood that the invention is not limited to the disclosed embodiments. To the contrary, it is intended to cover various modifications and similar arrangements (as would be apparent to those skilled in the art). Therefore, the scope of the appended claims should be accorded the broadest interpretation so as to encompass all such modifications and similar arrangements.

## Claims

1. A sustained release composition comprising of a polymer, a bioactive agent, and a release rate determined agent,
wherein the bioactive agent and the release rate determined agent are dispersed in the sustained release composition, and the release rate determined agent controls the release rate of the bioactive agent;
wherein the polymer is polyactide-co-glycolide (PLGA, the bioactive agent is olanzapine, and the release rate determined agent is vitamin E-TPGS;
and wherein the sustained release composition is produced by mixing the bioactive agents, the polymer and the release rate determined agent in an oil phase with an aqueous phase to form an emulsion, and solvents are subsequently removed from the emulsion to form hardened microparticles.

2. The sustained release composition as claimed in claim 1, wherein the release rate determined agent, is present in amounts of about 1 to 50 % by weight of the total weight of the sustained release composition.

3. The sustained release composition as claimed in claim 1, wherein the sustained release composition continually releases the bioactive agent.

4. The sustained release composition as claimed in claim 1, wherein the sustained release composition has an encapsulation rate of bioactive agent exceeding 70%.

5. The sustained release composition as claimed in claim 1, wherein the sustained release composition has a diameter exceeding 5 µm.

6. The sustained release composition as claimed in claim 1, wherein the sustained release composition is used for intramuscular or subcutaneous injection.

7. A method for manufacturing a sustained release composition, comprising:
(a) providing an oil phase comprising a bioactive agent, a polymer, and vitamin E-TPGS;
(b) providing an aqueous phase comprising a surfactant;
(c) mixing the oil phase with the aqueous phase to form an emulsion; and
(d) removing solvents to form hardened microparticles;
wherein the Polymer is polylactide-co-glycolide (PLGA and the bioactive agent is olanzapine.

8. The method as claimed in claim 7, wherein the solvent comprises methylene chloride, ethyl acetate, benzyl alcohol, acetone, acetic acid, propylene carbonate, dichloromethane, chloroform, 1,4-dioxane, dimethylformamide (DMF), dimethyl sulphoxide (DMSO), toluene, or tetrahydrofuran (THF).

9. The method as claimed in claim 7, wherein the surfactant comprises polyvinyl alcohol (PVA), NP-5, Triton x-100, Tween 80, PEG 200-800, sodium dodecyl sulfate (SDS), alcohol ethoxylates, alkylphenol ethoxylates, secondary alcohol ethoxylates, fatty acid ester, or alkyl polygylcosides.

10. The method as claimed in claim 7, wherein the release rate determined agents has a final concentration of about 1 to 50 % by weight.

## Patentansprüche

1. Zusammensetzung mit verlängerter Freisetzung, umfassend ein Polymer, ein bioaktives Mittel und ein freisetzungsgeschwindigkeitsbestimmendes Mittel,
wobei das bioaktive Mittel und das freisetzungsgeschwindigkeitsbestimmende Mittel in der Zusammensetzung mit verlängerter Freisetzung dispergiert sind und das freisetzungsgeschwindigkeitsbestimmende Mittel die Freisetzungsgeschwindigkeit des bioaktiven Mittels steuert;
wobei das Polymer Polylactid-co-glycolid (PLGA) ist, das bioaktive Mittel Olanzapin ist und das freisetzungsgeschwindigkeitsbestimmende Mittel Vitamin E-TPGS ist;
und wobei die Zusammensetzung mit verlängerter Freisetzung hergestellt wird durch Mischen der bioaktiven Mittel, des Polymers und des freisetzungsgeschwindigkeitsbestimmenden Mittels in einer Ölphase mit einer wässrigen Phase, um eine Emulsion zu bilden, und Lösungsmittel anschließend von der Emulsion entfernt werden, um gehärtete Mikroteilchen zu bilden.

2. Zusammensetzung mit verlängerter Freisetzung wie in Anspruch 1 beansprucht, wobei das freisetzungsgeschwindigkeitsbestimmende Mittel in Mengen von etwa 1 bis 50 Gew.-% des Gesamtgewichts der Zusammensetzung mit verlängerter Freisetzung vorhanden ist.

3. Zusammensetzung mit verlängerter Freisetzung wie in Anspruch 1 beansprucht, wobei die Zusammensetzung mit verlängerter Freisetzung kontinuierlich das bioaktive Mittel freisetzt.

4. Zusammensetzung mit verlängerter Freisetzung wie in Anspruch 1 beansprucht, wobei die Zusammensetzung mit verlängerter Freisetzung eine Einkapselungsrate des bioaktiven Mittels, welche 70 % übersteigt, aufweist.

5. Zusammensetzung mit verlängerter Freisetzung wie in Anspruch 1 beansprucht, wobei die Zusammensetzung mit verlängerter Freisetzung einen Durchmesser, welcher 5 µm übersteigt, aufweist.

6. Zusammensetzung mit verlängerter Freisetzung wie in Anspruch 1 beansprucht, wobei die Zusammensetzung mit verlängerter Freisetzung zur intramuskulären oder subkutanen Injektion verwendet wird.

7. Verfahren zur Herstellung einer Zusammensetzung mit verlängerter Freisetzung, umfassend:
(a) Bereitstellen einer Ölphase, umfassend ein bioaktives Mittel, ein Polymer und Vitamin E-TPGS;
(b) Bereitstellen einer wässrigen Phase, umfassend ein grenzflächenaktives Mittel;
(c) Mischen der Ölphase mit der wässrigen Phase, um eine Emulsion zu bilden; und
(d) Entfernen von Lösungsmitteln, um gehärtete Mikroteilchen zu bilden;
wobei das Polymer Polylactid-co-glycolid (PLGA) ist und das bioaktive Mittel Olanzapin ist.

8. Verfahren wie in Anspruch 7 beansprucht, wobei das Lösungsmittel Methylenchlorid, Ethylacetat, Benzylalkohol, Aceton, Essigsäure, Propylencarbonat, Dichlormethan, Chloroform, 1,4-Dioxan, Dimethylformamid (DMF), Dimethylsulfoxid (DMSO), Toluol oder Tetrahydrofuran (THF) umfasst.

9. Verfahren wie in Anspruch 7 beansprucht, wobei das grenzflächenaktive Mittel Polyvinylalkohol (PVA), NP-5, Triton x-100, Tween 80, PEG 200-800, Natriumdodecylsulfat (SDS), Alkoholethoxylate, Alkylphenolethoxylate, Sekundäralkoholethoxylate, Fettsäureester oder Alkylpolyglycoside umfasst.

10. Verfahren wie in Anspruch 7 beansprucht, wobei die freisetzungsgeschwindigkeitsbestimmenden Mittel eine Endkonzentration von etwa 1 bis 50 Gew.-% aufweisen.

## Revendications

1. Composition à libération prolongée comprenant un polymère, un agent bioactif, et un agent à vitesse de libération déterminée,
dans laquelle l'agent bioactif et l'agent à vitesse de libération déterminée sont dispersés dans la composition à libération prolongée, et l'agent à vitesse de libération déterminée contrôle la vitesse de libération de l'agent bioactif ;
dans laquelle le polymère est un polylactide-co-glycolide (PLGA), l'agent bioactif est l'olanzapine, et l'agent à vitesse de libération déterminée est la vitamine E-TPGS ;
et dans laquelle la composition à libération prolongée est produite par mélange des agents bioactifs, du polymère et de l'agent à vitesse de libération déterminée dans une phase huileuse avec une phase aqueuse pour former une émulsion, et les solvants sont ensuite éliminés de l'émulsion pour former des microparticules durcies.

2. Composition à libération prolongée telle que revendiquée dans la revendication 1, dans laquelle l'agent à vitesse de libération déterminée est présent dans des quantités d'environ 1 à 50 % en poids du poids total de la composition à libération prolongée.

3. Composition à libération prolongée telle que revendiquée dans la revendication 1, dans laquelle la composition à libération prolongée libère de façon continue l'agent bioactif.

4. Composition à libération prolongée telle que revendiquée dans la revendication 1, dans laquelle la composition à libération prolongée présente un taux d'encapsulation de l'agent bioactif dépassant 70 %.

5. Composition à libération prolongée telle que revendiquée dans la revendication 1, dans laquelle la composition à libération prolongée a un diamètre dépassant 5 *µ*m.

6. Composition à libération prolongée telle que revendiquée dans la revendication 1, dans laquelle la composition à libération prolongée est utilisée pour une injection intramusculaire ou sous-cutanée.

7. Procédé de fabrication d'une composition à libération prolongée, comprenant :
(a) la fourniture d'une phase huileuse comprenant un agent bioactif, un polymère, et de la vitamine E-TPGS ;
(b) la fourniture d'une phase aqueuse comprenant un tensioactif ;
(c) le mélange de la phase huileuse avec la phase aqueuse pour former une émulsion ; et
(d) l'élimination des solvants pour former des microparticules durcies ;
dans lequel le polymère est un polylactide-co-glycolide (PLGA) et l'agent bioactif est l'olanzapine.

8. Procédé tel que revendiqué dans la revendication 7, dans lequel le solvant comprend le chlorure de méthylène, l'acétate d'éthyle, l'alcool benzylique, l'acétone, l'acide acétique, le carbonate de propylène, le dichlorométhane, le chloroforme, le 1,4-dioxane, le diméthylformamide (DMF), le sulfoxyde de diméthyle (DMSO), le toluène, ou le tétrahydrofurane (THF).

9. Procédé tel que revendiqué dans la revendication 7, dans lequel le tensioactif comprend un polyalcool vinylique (PVA), NP-5, Triton x-100, Tween 80, PEG 200-800, le dodécylsulfate de sodium (SDS), des éthoxylates d'alcools, des éthoxylates d'alkylphénol, des éthoxylates d'alcools secondaires, un ester d'acide gras, ou des polyglycosides d'alkyle.

10. Procédé tel que revendiqué dans la revendication 7, dans lequel les agents à vitesse de libération déterminée ont une concentration finale d'environ 1 à 50 % en poids.
